# EUROPEAN PATENT SPECIFICATION

(11) **EP 2 806 809 B1**
(45) Date of publication and mention of the grant of the patent: **24.05.2017**
(21) Application number: 13702594.6
(22) Date of filing: 23.01.2013
(51) Int. Cl.: A61B 17/29, A61B 10/06, G01R 33/28, A61B 10/02, A61B 17/00

(54) **SURGICAL INSTRUMENT**
CHIRURGISCHES INSTRUMENT
INSTRUMENT CHIRURGICAL

(30) Priority: 25.01.2012 DE 102012201081
(43) Date of publication of application: 03.12.2014
(73) Proprietor: Maslanka, Herbert, 78532 Tuttlingen (DE); Ruprecht-Karls-Universität Heidelberg, 69117 Heidelberg (DE)
(72) Inventor: MASLANKA, Herbert, 78532 Tuttlingen (DE); STEEN, Henning, 69214 Eppelheim (DE); SEITZ, Sebastian, 69115 Heidelberg (DE)
(74) Representative: Weickmann & Weickmann PartmbB
(86) International application number: PCT/EP2013/051210
(87) International publication number: WO 2013/110640

(56) References cited:
- WO-A1-98/22022
- WO-A1-99/22797
- WO-A2-03/041785
- DE-C1- 10 043 787

## Description

The invention relates to a surgical instrument which can also be employed under the conditions of a magnetic resonance tomograph (MRT).

Conventional surgical instruments, such as biopsy forceps, in particular for myocardial biopsies, such as are known, for example, from DE 101 59 584 A1, comprise a forceps element having a forceps base and at least two forceps fingers guided such that they can move relative to one another on the forceps base, as well a manual actuation element for opening and closing the forceps element and an elongated, flexible actuation cable which connects the manual actuation element to the forceps element and has a tubular, flexible cable sheath connected to the forceps base and a flexible cable core which is guided displaceably in the cable sheath and is connected to the forceps fingers for opening and closing the forceps element.

In conventional biopsy forceps of this type, the components of the forceps element are made of non-rusting steel. The cable sheath of the actuation cable is constructed as spiral coiled spring and, like the cable core usually constructed from wire, is likewise made of metal, usually stainless steel.

The known biopsy forceps cannot be employed under the conditions of a magnetic resonance tomograph (nuclear spin tomograph) since the forceps element and the actuation cable generate artefacts in the imaging in which the image, in particular of the forceps element, depending on the location and imaging scale, does not correspond to the actual position of the forceps element in the patient's body and can completely overlap or falsify relevant areas. This makes evaluation of the image difficult. Furthermore, the forceps element and the actuation cable may heat up in the magnetic field.

The actuation cable of biopsy forceps of the type in question has a comparatively small diameter of, for example, 1.0 to 3 mm, but with a length of, for example, 50 cm to 1.4 m is comparatively long. Under the frequency conditions of the magnetic resonance tomograph, the elongated components of the actuation cable but also of the forceps element may heat up inductively and damage surrounding tissue, particularly if electromagnetic fields of the tomograph create resonance conditions on the actuation cable. Furthermore, because of the strong magnetic field of the tomograph, no ferromagnetic materials should be present, particularly on the forceps element or the actuation cable, in order to exclude the magnetic field of the tomography from undesirably deflecting the forceps element and the actuation cable.

Similar problems also result with other surgical instruments, for example instruments with the aid of which surgical components, such as occluders or vessel clips or stents, such as are known e.g. from WO 2010/130617 A1, are to be put in place under MRT conditions. In such instruments, the surgical component is held on a hook-like gripping device and is detached from the gripping device after placement in the body of the patient.

From WO 99/22797 A1 a guiding catheter system for delivering an elongated therapeutic or diagnostic device into a patient's left ventricle is known. The system includes a first guiding catheter with a shaped distal extremity configured to be aligned with or parallel to a longitudinal axis of the patient's left ventricle and a second guiding catheter slidably and rotatably disposed within an inner lumen of the first guiding catheter. The second guiding catheter also comprises an inner lumen which is configured to slidably receive an elongated therapeutic or diagnostic device such as a tissue ablation device. Each of the guiding catheters has a wall structure comprising an outer jacket containing a braided fibre reinforcement in a polymer matrix and an inner lubricious lining.

From WO 98/22022 A1 a medical interventional instrument for use in a magnetic resonance imaging system is known. The instrument comprises an outer cannula having a sharp tip and a tissue collection recess which opens into the interior of the cannula. An obturator insertable into and rotatable within the outer cannula allows excising tissue samples extending into the recess of the outer cannula. The outer cannula is made of braided carbon fibre material.

The object of the invention is a surgical instrument which can also be employed under the operating conditions of a magnetic resonance tomograph, in particular under real time conditions, and generates adequately image-true artefacts.

The invention is characterized by the features of claim 1 or claim 4.

The invention is based on a surgical instrument which comprises:
- a surgical tool having a tool base and a tool element guided such that it can move relative to the tool base,
- a manual actuation element for moving the tool element relative to the tool base, and
- an elongated, flexible actuation cable which connects the manual actuation element to the surgical tool and has a tubular, flexible cable sheath connected to the tool base and a flexible cable core which is guided displaceably in the cable sheath and is connected to the tool element for movement thereof relative to the tool base.

The object explained above is achieved according to the invention in that at least the tool element of the surgical tool, but in particular also the tool base thereof, is made substantially of a non-ferromagnetic or substantially non-ferromagnetic metal or of ceramic or of plastic, and in that the cable sheath and the cable core are each made of an electrically non-conducting or substantially non-conducting composite material in which a plurality of reinforcing threads are embedded in a plastics matrix.

It has been found that conventional materials which can be employed in magnetic resonance tomographs as a rule do not meet the requirements imposed on the actuation cable of a surgical instrument, in particular a gripper instrument. In order to be able to reliably actuate the surgical tool arranged on the distal end of the actuation cable, it must be possible to exert a force of from 20 to 40 N, possibly even more than 100 N, based on the cable sheath, many times on the surgical tool via the cable core. Since the actuation cable is comparatively thin and long, several times this force, for example 100 to 300 N, must be exerted on the cable core, based on the cable sheath, at the manual actuation element in order to be able to overcome the friction in the actuation cable, especially since tangential losses of force occur due to bends in the actuation cable during operation. It has furthermore been found that conventional plastics materials such as can be employed in a magnetic resonance tomograph are not adequately resistant to elongation, so that they are only inadequately elongation-resistant under the force conditions mentioned. Adequately high actuation forces on the tool element lead in this manner to excessively long actuation paths on the manual actuation element in the case of conventional MRT-suitable plastics materials. It has been found, surprisingly, that the abovementioned problems can be overcome if the cable sheath and the cable core are each made of an electrically non-conducting or substantially non-conducting composite material in which a plurality of reinforcing threads are embedded in a plastics matrix. The reinforcing threads increase the tensile and compressive strength of the cable sheath and cable core even under the operating conditions of the magnetic resonance tomograph. The cable sheath has a tubular shape. It goes without saying that the tube forming the cable sheath optionally can also have several lumina separated from one another.

Since the tool element and optionally also the tool base is/are substantially made of non-ferromagnetic metal, that is to say of diamagnetic or paramagnetic material or of ceramic or of plastic, they can be exposed to the strong magnetic field of the tomograph without being deflected or displaced by the magnetic field. Titanium, in particular, is suitable, but platinum, gold, silver, copper or aluminium or alloys based on such metals are also suitable. If in particular the tool element is made of metal or ceramic, it is adequately resistant to wear and can optionally be provided with cutting edges or the like for severing tissue. The cable sheath and the cable core are electrically non-conducting and accordingly do not heat up under the operating conditions of the tomograph, that is to say they can also be employed with tomographs having a particularly high magnetic field strength of more than 3 T. It is essential that the cable sheath and the cable core are made of a composite material in which reinforcing threads are embedded in a plastics matrix and impart to the cable sheath and the cable core adequate tensile and compressive rigidity and also adequate torsional rigidity in order, for example, to be able to open and close but also rotate a forceps tool manually by means of the manual actuation element with a definable use of force, for example for the removal of biopsy samples.

In a preferred embodiment, the surgical tool is constructed as a holding tool having a gripping device which forms the tool element and is guided such that it can move relative to the tool base forming a holding base, the manual actuation element for opening and closing the holding tool via the cable core being connected to the gripping device.

The tool element or the gripping device can be a coupling piece, in particular a hook or the like, as is known for putting in place an occluder or a vessel clip or a stent. Preferably, the surgical instrument is constructed as a gripper instrument, in which the surgical tool is constructed as a forceps tool and comprises, on a forceps base connected to the cable sheath, at least two forceps fingers which are guided such that they can move relative to one another on the forceps base. The forceps fingers in turn are connected to the cable core for opening and closing the forceps tool.

It goes without saying that the forceps tool of the gripper instrument can be configured in various ways. Thus, for example, the forceps fingers can be constructed as spoons which are linked to the forceps base and connected to the cable core via a steering gear, for example in the form of a scissors gear, as is known, for example, from DE 101 59 584 A1. Alternatively, however, the forceps fingers can also be constructed, as is usual in the case of holding forceps, such that they are spring-mounted radially to the cable sheath and firmly connected to the cable core, so that by means of the cable core they can be drawn into an end region of the cable sheath forming the tool base for closing the forceps tool. When the forceps fingers are pushed out of this end region, they spread away from one another to open the forceps tool. The gripper instrument can also be a collecting basket or the like which spreads out in a sprung manner. Alternatively, however, the gripper instrument can also be a collecting loop which is connected to the cable core and spreads out automatically when it is pushed out of the tool base.

At least the tool element, such as the forceps fingers, but where appropriate also the tool base or the forceps base, are preferably made of titanium or a titanium alloy in order to achieve artefact imaging of the holding element which is as far as possible true to scale. However, other paramagnetic or diamagnetic metals, such as platinum, gold, silver, copper or aluminium or alloys thereof, are also suitable.

The overall dimensions of the surgical tool and/or of the manual actuation element, where this is made of electrically conducting material, in particular metal, are preferably less than 10 cm, in particular less than 6 cm and preferably less than 3 cm. Electromagnetic resonances under the operating conditions of the tomograph and therefore heating up of the metal parts by the electromagnetic field can be reliably avoided in this manner. Although the cable sheath and/or the cable core is/are preferably made overall of electrically non-conducting or substantially non-conducting material, adhering to the above dimension limits allows the use of segmented electrically conducting components which are electrically insulated from one another at least between the segments. This allows e.g. the use of electrically conducting reinforcing thread segments, such as carbon reinforcing thread segments.

The cable sheath must transmit in a reliable and largely non-elastic manner tensile and compressive forces as well as torsional forces from the manual actuation element to the tool element, such as the forceps tool. Nevertheless, it must be ensured that the actuation cable is adequately flexurally elastic. In a preferred embodiment, these properties are achieved in an optimum manner, in spite of the comparatively small diameter of the cable sheath, if the reinforcing threads of the composite material of the cable sheath are coiled around the central axis thereof. In particular, in this embodiment the composite material of the cable sheath comprises reinforcing threads coiled in opposite directions around the central axis thereof in order to obtain properties which correspond to one another in both torsional rotation directions. The reinforcing threads coiled in opposite directions can be arranged in two separate winding layers lying one on top of the other, in which they are fixed by the plastics matrix. Particularly high tensile and compressive and torsional rigidity is achieved, however, if the reinforcing threads form a woven structure, in particular in the form of a woven tube. A woven structure is also to be understood as meaning a woven-like structure, such as a knitted or braided or stitched structure. It is essential for such a woven structure that reinforcing threads run essentially symmetrically to a generatrix of the cable sheath, so that a torsional strength results which is symmetrical around the central axis in both directions of rotation.

In a preferred embodiment, the cable sheath comprises a multi-layered structure of non-conducting or substantially non-conducting plastics material, in particular having a tubular inner sheath layer optionally comprising several lumina and an outer sheath layer, surrounding the inner sheath layer, made of such a plastics material, the reinforcing threads being arranged between the inner and the outer sheath layer, that is to say forming, for example, a middle layer made of a woven tube. In such a cable sheath, the inner sheath layer and the outer sheath layer can be formed from plastics materials of different hardness and/or flexural elasticity. In particular, the inner sheath layer can be harder or more rigid than the outer sheath layer, which leads to a reduction in the loss of actuation force.

The cable core runs freely displaceably in the cable sheath and accordingly has an external diameter of the order of, for example, 0.5 to 1 mm. Nevertheless, the cable core must also have an adequate tensile and compressive rigidity, so that the length of the cable core changes only insignificantly under load. Under the given dimensional conditions, the reinforcing threads of the cable core preferably run substantially in the longitudinal direction of the cable core. The reinforcing threads of the cable core can be constructed as thread sections which, however, are preferably in each case longer than 3 cm. Preferably, the reinforcing threads of the cable core extend in one piece over the entire length thereof.

The cable core is preferably constructed as more rigid to tensile and compressive stress than the cable sheath. The cable core accordingly should have a high modulus of elasticity of at least 2,000 MPa, measured in accordance with ASTM D 638/DIN EN ISO 527, and/or a ball indentation hardness of at least 120 MPa (DIN EN ISO 2039-1). The yield stress, measured in the tensile test according to DIN EN ISO 527, should be at least 60 MPa. The cable core should withstand forces of at least 100 N, but more preferably at least 200 N, without or without noticeable plastic deformation.

Suitable plastics for the plastics matrix of the cable core are epoxy resin and/or a plastic from the group of polyamides, in particular PA 6 or PA66, and/or the group of polyimides (PI) and/or polyetherimides (PEI), and/or the group of polyamide-imides (PAI) and/or the group of polyaryletherketones (PAEK) and/or of polyetheretherketones (PEEK) and/or polyetherketones (PEK), and/or the group of polyphenylene sulphides (PPS), and/or the group of polysulfones (PSU) in particular polyarylsulfones and/or of polyphenylsulfones (PPSU) and/or of polyethersulfones (PES), and/or the group of liquid crystal polymers (LCP). The plastics matrix should comprise at least one of these plastics.

The plastics matrix of the composite material of the cable sheath preferably comprises plastics which are less hard and/or more flexurally elastic than the plastics matrix of the cable core. Suitable plastics are, for example, polyamide (PA), polyethylene (PE), polypropylene (PP), polyurethane (PU), polytetrafluoroethylene (PTFE), perfluoroethylene/propylene plastic (FEP), perfluoroalkoxyalkane (PFA) or polyether block amide (PEBAX). The plastics matrix of the cable sheath should comprise at least one of these plastics. In a multi-layered construction of the cable sheath, the inner sheath layer is preferably made of PA or PPSU, while the outer sheath layer is made of a plastics material which, in contrast, is less hard or more flexible, for example PEBAX. The inner sheath layer is preferably doped with a lubricant, in particular BaSO₄, in order to improve the slip properties of the cable sheath. Doping with BaSO₄ moreover improves the visibility of the actuation cable in the magnetic resonance tomograph without noticeable artefact formation.

The reinforcing threads of the composite material of the cable sheath and/or of the cable core are preferably aramid fibres or glass fibres or polyester fibres or ceramic fibres or fibres of liquid crystal polymer. In each case a plurality of such fibres can be combined or twisted into threads for easier handling. Reinforcing threads of glass fibres improve the tensile and shear values of the cable core to a particularly high degree. In order also to improve the breaking strength, the cable core is preferably reinforced with a mixture of reinforcing threads of different material, in particular of glass fibres and aramid fibres, embedded in the plastics matrix. In this connection, PEEK has proved to be particularly suitable as the material of the plastics matrix.

Where the materials of the surgical or forceps tool and of the actuation cable are electrically non-conducting, they can form artefacts which are not adequately detectable in magnetic resonance imaging, depending on the susceptibility (magnetisability) thereof. In order to facilitate evaluation of the image, the cable sheath and/or the cable core can carry or comprise at least regions of marking material, for example metal, in particular in the form of particles of this material, which has a susceptibility which differs from water. The marking can be in the form of rings, which can optionally also be made completely of metal and can also be used at predetermined intervals, in the nature of a measuring scale, for length measurement under MRT conditions. The measuring scale can be constructed, for example, as a cm scale and can optionally be provided at intervals on the actuation cable with an inscription. The zero point of the measuring scale is expediently at the distal end, i.e. the tip of the tool element. Such a marking is of advantage in particular in the case of non-metallic tool elements, for example forceps fingers, for example made of ceramic or plastic, but also in the case of the electrically non-conducting actuation cable, in particular the cable sheath thereof. In the case of tool elements constructed as a gripping device, it may be of advantage for only some of the forceps fingers which can move relative to one another be coated or doped with marking material, in order to generate different MRT artefacts of the individual forceps fingers in this manner. The open or closed state of the forceps tool can be rendered visible in this manner.

In order to be able to insert the surgical instrument more easily into the body of the patient, it is advantageous for the actuation cable to be more flexible in configuration in a limited region close to the surgical tool than in its remaining length leading to the manual actuation element. In a preferred embodiment in which the reinforcing threads of the cable sheath surround the central axis in the form of a coil, this can be achieved by coiling the reinforcing threads in the more flexible region with a lower pitch than in the region of the cable sheath lying between this region and the manual actuation element. The diameter of the cable core is also expediently chosen to be smaller in the flexible region than in the residual length of the cable core following towards the manual actuation element.

The surgical instrument is expediently inserted into the body of the patient via an MRT-capable catheter. The catheter preferably has a distal end which can be controlled in its degree of bending. The controllable end can, for example, be curved manually before insertion into the body or, however, in the inserted state can be controlled in its curvature from a manual actuation device via a drawing wire guided in the catheter.

In a preferred embodiment, the curvature of a region of the actuation cable adjacent to the surgical tool can be controlled from the manual actuation element, as is known for conventional biopsy forceps from DE 36 41 935 A1. This facilitates the insertion of the surgical instrument into the body of the patient and placement of the surgical tool in a desired position. In the case of a surgical instrument which can be employed under MRT conditions, the curvature of the end region of the actuation cable adjacent to the surgical tool can also be controlled in a constructionally simple manner if a further, flexible cable core of electrically non-conducting or substantially non-conducting material is guided displaceably in the cable sheath, the end of which cable core adjacent to the tool base is fixed eccentrically to a central axis of the cable sheath on the tool base or close to the tool base on the cable sheath. The further cable core is also displaceable by means of a control unit of the manual actuation element. If the further cable core is pulled out of the cable sheath, because of its eccentric connection of its end adjacent to the surgical tool, it curves the end region of the cable sheath. In the position pulled out of the cable sheath, the further cable core can expediently be stopped at the manual actuation element.

The embodiment explained above of a flexible actuation cable having an end region which can be controlled in its curvature can be employed even if the actuation cable has a comparatively small diameter. In order to limit the region of controllable curvature in a predetermined manner and to ensure a comparatively large angle of curvature in this region, in a preferred embodiment, for formation of the region of controllable curvature, the cable sheath has non-uniform flexurally elastic properties transversely to the central axis in a section of its length adjacent to the tool base viewed in the circumferential direction, and is more flexible towards the eccentrically fixed end of the further cable core than outside this region. The flexible properties of the curvature region moreover lessen the transverse forces exerted by the further cable core on the cable sheath.

The more flexible properties of the curvature region can be realised in various ways. On the one hand, the plastics matrix of the curvature region can be made of a more elastic or more flexible plastics material than outside the curvature region. However, the bending properties can also be influenced by suitable choice of the reinforcement of the cable sheath. For example, as already explained above, the pitch of the reinforcing threads coiled around the central axis can be smaller in the curvature region than outside the curvature region. In addition or alternatively, on the side of the central axis facing away radially from the eccentrically fixed end of the further cable core, in the region of controllable curvature, the cable sheath can be provided with a reinforcement extended in the direction of the central axis. In this manner, on its side exposed to tension during curving, the cable sheath is stiffened by the additional, longitudinally running reinforcement. In addition or alternatively, however, in the region of controllable curvature on the side of the central axis lying radially to the eccentrically fixed end of the further cable core, the cable sheath can also be made of a softer material, at least in a part-region, than at least in a part-region on the diametrally opposite side. Curving of the cable core is also facilitated by this measure.

The curvature properties of the cable sheath can additionally or alternatively also be influenced, however, by its design. In a preferred embodiment, in the region of controllable curvature the cable sheath has, on the side lying towards the eccentric end of the further cable core with respect to the central axis, a plurality of recesses arranged at intervals from one another in the direction of the central axis. The recesses are expediently grooves or cavities extending in the circumferential direction over a part-length of the circumference. The recesses are preferably provided on the outer circumference of the cable sheath. In the region of controllable curvature, the cable sheath is expediently sheathed with a flexible protective tube, which smoothes the surface of the cable sheath. Where the plastics matrix of the cable sheath, as explained above, is constructed in several layers, it goes without saying that the recesses or cavities can also be provided on the inner layer of the plastics matrix.

The cable core connected to the surgical tool can have a smaller diameter in the controllable curvature region than outside this region, in order to be able to curve the actuation cable overall more easily in this region. The further cable core can accordingly also have a smaller diameter in the controllable curvature region than outside the region. In the transition region between the smaller and the larger diameter, the cable cores expediently taper conically, in order to reduce the risk of breaking in the transition region.

The manual actuation element may also be exposed to the strong magnetic field of the tomograph, which may possibly also lead to a heating up of the manual actuation element, especially if it comprises metal parts. In order to prevent excessive heating up of the manual actuation element, the manual actuation element is expediently made of a non-magnetic, electrically conducting material, in particular plastic, at least in its manually accessible outer surfaces.

Embodiments of the invention are explained in more detail in the following with the aid of a drawing.
Figure 1 shows a side view of biopsy forceps which can also be employed under the operating conditions of a magnetic resonance tomograph;
Figure 2 shows a partly broken open detail view of a forceps element of the biopsy forceps in the region of the arrow II in Figure 1;
Figure 3 shows a section view of an actuation cable, seen along a line III-III in Fig. 2;
Figures 4 and 5 show side views of holding forceps such as can be employed instead of the forceps element of the biopsy forceps of Figures 1 to 3;
Figure 6 shows a side view of a holding loop for a surgical instrument having an actuation cable and a manual actuation element according to Figures 1 to 3;
Figure 7 shows a side view of a holding basket for a surgical instrument having an actuation cable and a manual actuation element of Figures 1 to 3;
Figures 8 and 9 show perspective diagrams of coupling tools for surgical instruments having an actuation cable and a manual actuation element according to Figures 1 to 3;
Figure 10 shows a partly broken open detail view of biopsy forceps similar to the biopsy forceps of Figures 1 to 3, but on which the actuation cable adjacent to the forceps element ends in a region of controllable curvature;
Figure 11 shows a partly broken open detail diagram of the actuation cable in the region of controllable curvature;
Figure 12 shows a side view of the actuation cable from Figure 11 in the curved state;
Figure 13 shows a side view of the actuation cable through the region of controllable curvature, seen along a line XIII-XIII in Figure 11 and
Figures 14 and 15 show detail views of variants of the actuation cable of controllable curvature.

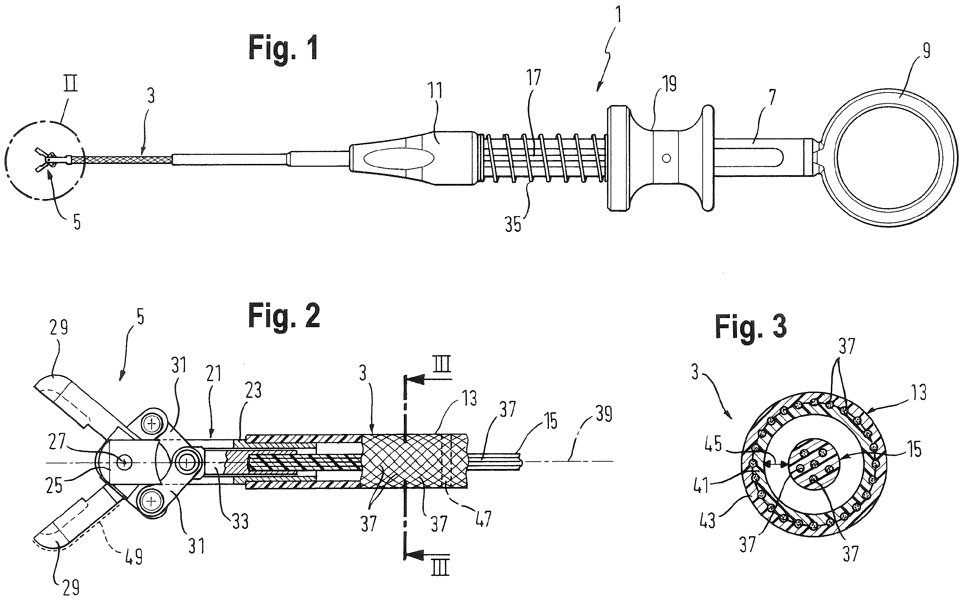

The surgical biopsy forceps shown in Figure 1, in particular for myocardial biopsies, comprise a manual actuation element 1 which is connected via a flexible, comparatively thin but long actuation cable 3 to a surgical tool or holding element in the form of a forceps tool 5 to form a unit. The manual actuation element 1 comprises a guide shaft 7 which carries a thumb ring 9 on its end away from the patient. A flexible cable sheath 13 (Figure 2) of the actuation cable 3, explained in more detail below, is fixed by means of a sheath 11 on the end of the guide shaft 7 on the patient side. The cable sheath 13 is tubular and surrounds a flexible cable core 15, which is guided displaceably in the cable sheath 13. The cable core 15 is connected via an extension piece 17 to an index finger-middle finger sliding grip 19 guided displaceably on the guide shaft 7. The forceps tool 5 comprises a forceps or tool base 21 which is constructed as a forked tube and is fixed, for example stuck, onto a tube section 23 on the end of the cable sheath 3 on the patient side, and on its side away from the cable sheath 3 carries two fork halves 25 diametrally opposite one another. On the fork halves 25, only one of which is shown in Figure 2, two forceps fingers 29, here spoon-shaped, forming a gripping device, are mounted swivellably relative to one another and to the tool base 21 on a diametrally running axis 27. The fork halves 25 are coupled in an articulated manner via a scissors lever 31 to an end piece 33 of the cable core 15 on the patient side. When the thumb ring 9 is moved away from the index finger-middle finger sliding grip 19, the forceps tool 5 opens. The thumb grip 9 and the index finger-middle finger sliding grip 19 are moved towards one another by a pressure spring 35 clamped between the guide shaft 7 or the sheath 11 and the patient side of the index finger-middle finger sliding grip 19, so that the forceps tool 5 closes. The closing force can be increased manually, if appropriate, via the manual actuation element 1.

For MRT-guided biopsies, in particular myocardial biopsies, the dimensions of the forceps tool 5 are comparatively small. For example, the forceps tool 5 conventionally has a diameter of less than 3 mm at a length of between 6 and 15 mm. The actuation cable 3 in its turn is comparatively thin having an external diameter of from, for example, 1.0 to 3 mm, but comparatively long, for example, between 50 cm to 1.4 m. In spite of these dimensional conditions, both the cable sheath 13 and the cable core 15 must have tensile and compressive rigidity in order to be able to transmit the forces required for opening and closing the forceps tool 5 without a noticeable, load-related change in length. The actuation cable 3 and in particular the cable sheath 13 thereof must moreover be substantially torsionally rigid in order to be able to rotate the forceps tool 5 in a defined manner around the axis of the actuation cable 3. In spite of these rigidity requirements, the actuation cable 3 must be adequately flexible.

In conventional biopsy forceps, the cable sheath is constructed as a metallic coiled spring, of which the windings adjacent to one another can transmit adequately high tensile and compressive forces in a torsionally rigid manner. The cable core is usually made of a metal wire of solid cross-section. In a magnetic resonance tomograph, the forceps tool and the actuation cable of conventional biopsy forceps generate image artefacts which are not true to scale and make evaluation of the image difficult. Furthermore, the metal parts of the actuation cable heat up in the electromagnetic field of the tomograph. Where the material of the forceps tool and of the actuation cable has ferromagnetic properties, these components are deflected in an undesirable manner in the magnetic field of the tomograph.

In order to avoid the disadvantages explained above, both the cable sheath 13 and the cable core 15 are produced from an electrically substantially non-conducting, flexible composite material, in which in each case a plurality of reinforcing threads 37 running along the actuation cable 3 are embedded in a plastics matrix. In the composite material of the cable sheath 13, the reinforcing threads 37 run, as shown in Fig. 2, coiled in opposite directions around the central axis 39 of the cable sheath 13. The reinforcing threads 37 are preferably combined to form a braided structure or woven structure in the form of a tube, but also optionally to form a knitted structure or stitched structure, or, however, are wound around the central axis 39 in at least two layers lying radially one on top of the other.

As shown in Fig. 3, the cable sheath 13 is constructed in several layers and comprises a tubular inner sheath layer 41 of electrically non-conducting or substantially non-conducting plastics material and a tubular outer sheath layer 43, surrounding the inner sheath layer 41, of a likewise electrically non-conducting or substantially non-conducting plastics material. The reinforcing threads, here the tubular braided structure or woven structure of reinforcing threads 37, is arranged between the inner sheath layer 41 and the outer sheath layer 43. The two layers 41, 43 are firmly connected to one another and anchor the reinforcing threads 37. In the cable core 15, the reinforcing threads 37 run at least approximately parallel to the direction of the central axis 39.

The cable core 15 runs in the cable sheath 13 with radial play 45. The play 45 should, on the one hand, be a small as possible in order to keep path losses between the actuation path of the manual actuation element 1 and the actuation path of the forceps tool 5 as low as possible during actuation of the manual actuation element 1. On the other hand, diameter tolerances which the play must compensate may result during production of the cable core 15 and the cable sheath 13. Based on half the diameter difference shown in Fig. 3, the play 45 should not be greater than one quarter of the internal diameter of the cable sheath 13.

Preferably, the reinforcing threads 37 extend integrally substantially over the entire length of the actuation cable 3. It goes without saying, however, that the reinforcing threads can also extend merely over a part-region of the total length of the actuation cable, since they are firmly connected to one another via the plastics matrix. The reinforcing threads 37 can be constructed, for example, as aramid fibres or glass fibres or polyester fibres or ceramic fibres or fibres of liquid crystal polymer. Glass fibres have proved to have a particular tensile and shear strength especially for the cable core 15. In order to reduce the risk of breaking of the glass fibres, the glass fibres are preferably embedded in the plastics matrix as a mixture with aramid fibres. Electrically conducting fibres, for example carbon fibres, can optionally also be used to a certain extent if these fibres are segmented and the segments are electrically insulated from one another and have a length of less than 10 cm, in particular less than 6 cm and preferably less than 3 cm.

It has proved favourable for the reinforcing threads 37 in each case to be made of a very large number of reinforcing fibres, the fibre diameter of which in each case is of the order of 1/100 mm or thinner. A very large number of such fibres can be bundled or twisted into a thread, the diameter of which is between about 0.1 to 0.15 mm. The reinforcing of the cable core 15 is made of a plurality of such reinforcing threads, which can be embedded in the plastics matrix by coextrusion. The reinforcing threads 37 of the cable sheath 13 are expediently applied to the inner sheath layer 41, for example by means of a tubular braiding machine, before the outer sheath layer 43 is applied to this composite, for example likewise by coextrusion.

The material of the plastics matrix of the cable sheath 13 can be polyamide (PA), polyethylene (PE), polypropylene (PP), polyurethane (PU), polytetrafluoroethylene (PTFE), perfluoroethylene-propylene plastic (FEP), perfluoroalkoxyalkane (PFA) or a polyether block amide (PEBAX). The plastics matrix should comprise at least one of these plastics materials as the highest content. The inner sheath layer 41 expediently is made of PA or PPSU. For the outer sheath layer 43, PEBAX has proved to be suitable. In order to achieve a firm connection between the sheath layers 41, 43, the materials of the plastics matrix are preferably thermoplastics. To improve the slip properties and to improve visibility in the magnetic resonance tomograph without substantial formation of artefacts, the plastics matrix is expediently doped with BaSO₄ or another lubricant.

The material of the plastics matrix of the cable core 15 is preferably epoxy resin or a plastic from the group of polyamides, in particular PA 6 or PA66, and/or the group of polyimides (PI) and/or polyetherimides (PEI), and/or the group of polyamide-imides (PAI), and/or the group of polyaryletherketones (PAEK) and/or of polyetheretherketones (PEEK) and/or of polyetherketones (PEK), and/or the group of polyphenylene sulphides (PPS), and/or the group of polysulfones (PSU) in particular polyarylsulfones and/or of polyphenylsulfones (PPSU) and/or of polyethersulfones (PES), and/or the group of liquid crystal polymers (LCP). The plastics matrix should comprise at least one of these plastics materials as the highest content. PEEK and PPSU have proved to be particularly suitable.

The cable core should have a ball indentation hardness of more than 120 MPa (measured in accordance with DIN EN ISO 2039-1) and an E modulus of at least 2,000 MPa (measured in accordance with ASTM D 638, DIN EN ISO 527). The yield stress of the material of the plastics matrix of the cable core 15 should be at least 60 MPa (measured in accordance with DIN EN ISO 527). Plastics materials which meet these requirements are e.g. PEEK and PPSU. The ball indentation hardness of the plastics materials of the inner sheath layer 41 and of the outer sheath layer 43, like the E modulus of these materials and the yield stress thereof, can be lower. For the material of the reinforcing threads but also of the plastics matrix, it is essential that they should not lose their strength under the operating conditions of the magnetic resonance tomograph. The material of the reinforcing threads and of the plastics matrix must therefore have temperature-constant or substantially temperature-constant expansion properties at least up to 60 °C, but preferably up to 130 °C, that is to say have no or no noticeable losses in strength. The cable core should be able to absorb forces of at least 100 N, more preferably at least 200 N, without plastic deformation.

It goes without saying that the material of the cable core and of the cable sheath, like that of the surgical tool and of the manual actuation element, must also be able to withstand relatively high temperatures in order to be able to sterilise or autoclave the instrument.

At least the forceps fingers 29 of the forceps tool 5 are preferably made of titanium or a titanium alloy in order to achieve artefacts which are as image-true as possible. However, other paramagnetic or diamagnetic materials, such as platinum, gold, silver, copper or aluminium or alloys of these metals, are also suitable. Since the forceps tool 5 is made of metal, it is visible in the tomograph, so that the biopsy forceps can be put in place. Because of the small dimensions of the forceps tool 5, the risk of heating up in the electromagnetic field of the tomograph is low. Individual components of the forceps tool 5, such as the axis 27, can therefore also be produced from less expensive materials, such as non-rusting steel having non-magnetic properties, if artefacts generated in the image by such components can be tolerated.

While the components of the forceps tool 5 are visible in the tomograph, the actuation cable 3 does not image detectably or images such that it is only poorly detectable. It has therefore proved favourable for the actuation cable 3, in particular the cable sheath 13 thereof, to be doped or coated with metallic particles at least in regions, or for metallic marking rings, as indicated at 47, to be applied as visible marks at predetermined intervals on the actuation cable 13. The marking rings 47 can be made of solid material or in turn of regions doped with metal. Such visible marks can also be used as scale marks.

The two forceps fingers 29 can be constructed such that they generate different artefacts in the tomograph. The open or closed state of the forceps tool 5 can also be shown in this manner. The two forceps fingers 29 can be made of different material or be doped or coated differently with marking material, as is indicated at 49 in Figure 2. Alternatively, one of the forceps fingers 29 can also carry a small insert of an artefact-forming material.

However, in order to rule out heating up of metallic or electrically conducting components in the electromagnetic field of the tomograph, the dimensions of these components should be shorter than 10 cm, in particular shorter than 6 cm and preferably shorter than 3 cm. This also applies to any metallic components of the manual actuation element 1, such as the extension piece 17, which is conventionally constructed as a metal rod, or the pressure spring 35, especially if the biopsy forceps are used in an open magnetic resonance tomograph in which the manual actuation element 1 may also be exposed to the magnetic field. The manual actuation element 1, at least in its manually accessible outer surfaces, such as the thumb ring 9, the guide shaft 7 and the index finger-middle finger sliding grip 19, is expediently made of a non-magnetic, electrically non-conducting material, in particular plastic. If metal parts, such as the extension piece 17 or the pressure spring 35, are provided, the dimensions are chosen to be smaller than 10 cm and preferably smaller than 6 cm.

Variants of the surgical instrument explained above with the aid of Figures 1 to 3 are explained in the following. Components with the same action are designated with the same reference numerals as in Figures 1 to 3 and are provided with a letter for differentiation. For explanation of the construction and the mode of action, including of any variants, reference is made to the above description of Figures 1 to 3.

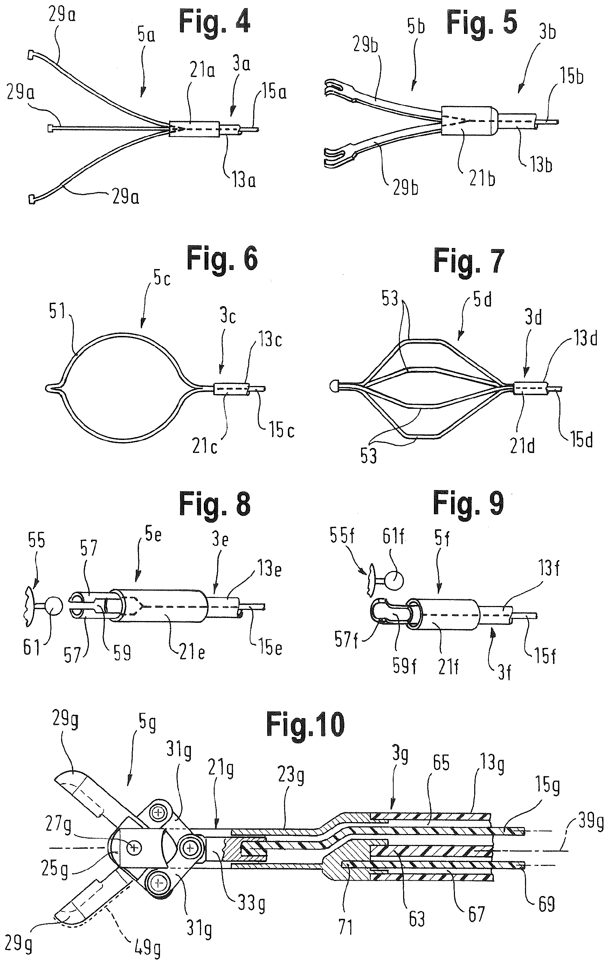

Figure 4 shows a variant of a surgical instrument which differs from the biopsy forceps of Figures 1 to 3 essentially only in that the surgical tool connected via the actuation cable 3a to the manual actuation element (not shown in more detail) is constructed as three-fingered holding forceps 5a. The holding forceps 5a have a sheath-like tool base 21 a which is connected to the cable sheath 13a and into which the forceps fingers 29a which spread out in an elastically sprung manner radially to the central axis can be drawn by means of the cable core 15a connected to the forceps fingers 29a. The tool base 21 a squeezes the forceps fingers 29a towards one another during drawing in and closes the forceps tool 5a in this manner. Reference is expressly made to the description of the actuation cable 3 and of the manual actuation element 1 of Figures 1 to 3 for the construction and for the mode of action of the actuation cable 3a and of the manual actuation element and of any variants.

Figure 5 shows a surgical instrument having two-fingered holding forceps 5b which are connected via the actuation cable 3b to the manual actuation element, in turn not shown in more detail. The holding forceps 5b have two forceps fingers 29b which spread out radially in a sprung manner and can be drawn into a likewise sheath-like forceps base 21 b with the cable core 15b. The forceps base 21 b is connected to the cable sheath 13b and squeezes the forceps fingers 29b towards one another during drawing in for closing the forceps tool 5b.

The forceps fingers 29a, 29b shown in Figures 4 and 5 are made of non-ferromagnetic material, for example titanium or platinum, gold, silver, copper, aluminium or an alloy thereof, it also being possible here for one of the forceps fingers to be doped or coated or equipped with marking material for visualisation of the open or closed state of the forceps tool 5a or 5b, as has been explained for the coating 49 with the aid of Figure 2.

Figure 6 shows a surgical instrument having a collecting loop 5c which is connected via a flexible actuation cable 3c to a manual actuation element (not shown in more detail). The collecting loop 5c has a loop 51 which spreads out in a sprung manner and can be drawn by means of the manual actuation element via a cable core 15c guided displaceably in a cable sheath 13c into a sheath-like end region 21 c of the cable sheath 13c forming a tool base. When drawn into the cable sheath 13c, the loop 51 is closed. The actuation cable 3c and the manual actuation element correspond to components 3 and 1 respectively of the surgical instrument of Figures 1 to 3, including the variants explained there. The loop 51 is made of non-ferromagnetic material, in particular titanium or platinum, gold, silver, copper, aluminium or an alloy thereof, but can also be made of an elastic plastics material.

Figure 7 shows a surgical instrument having a surgical tool 5d which is constructed as a collecting basket and can be drawn via a cable core 15d, guided displaceably in a cable sheath 13d of an actuation cable 3d, by means of a manual actuation element (not shown in more detail) into an end region 21 d of the cable sheath 13d forming a tool base. The collecting basket 5d has several basket struts 53 distributed in the circumferential direction which spread out radially and elastically and, when drawn into the end region 21d of the cable sheath 3d, are squeezed towards one another and therefore close the collecting basket 5d. The basket struts 53 in turn are made of titanium or platinum, gold, silver, copper, aluminium or an alloy thereof or of electrically non-conducting, elastic plastics material, that is to say are not ferromagnetic. The actuation cable and the manual actuation element otherwise correspond to components 3 and 1 of the instrument explained with the aid of Figures 1 to 3.

Figure 8 shows a surgical instrument for putting in place a surgical component 55, for example an occluder, a vessel clip or a stent, which can be positioned in the body of a patient by means of the surgical instrument and can be uncoupled from the instrument there. The surgical instrument comprises a coupling tool 5e with two coupling hooks 57 opposite one another, which between them form an accommodating space 59 which tapers towards the free end and is for accommodation of a coupling head 61 standing away from the surgical component 55. The coupling hooks 57 are connected to a cable core 15e which can be displaced in the cable sheath 13e of an actuation cable 3e and via which, by means of a manual actuation element (not shown in more detail), the coupling hooks 57 can be drawn into a sheath-like tool base 21 e connected to the cable sheath 13e. The coupling head 61 inserted in the accommodating space 59 is locked within the tool base 21 e by this and comes free when the coupling hooks 57 are pushed out. The coupling hooks 57 and the tool base 21 e are made of non-ferromagnetic material, in particular titanium or platinum, gold, silver, copper, aluminium or an alloy thereof or of electrically non-conducting plastic. The actuation cable 3e and the manual actuation element otherwise correspond to components 3 and 1 of the instrument of Figures 1 to 3, including the variants explained for these there.

Figure 9 shows a surgical component 55f which is provided with a coupling head 61f and can be positioned in the body of a patient by means of a surgical instrument and released there. The instrument in turn comprises a coupling tool 5f which is connected via an actuation cable 3f comprising cable sheath 13f and cable core 15f to a manual actuation element (not shown in more detail). In contrast to the coupling tool 5e of Figure 8, the coupling tool 5f has only one coupling hook 57f which, together with a sheath-like coupling base 21f connected to the cable sheath 13f, limits an accommodating space 59, which tapers towards the free end of the coupling hook 57f, for the coupling head 61f. When drawn into the coupling base 21f, the coupling head 61f is coupled to the instrument. When the coupling hook 57f is pushed out of the coupling base 21f, the coupling head 61f is released.

The coupling hook 57f and optionally the tool base 21f, like the coupling head 61f, are made of non-ferromagnetic material, for example titanium or platinum, gold, silver, copper, aluminium or an alloy thereof or of electrically non-conducting plastic. The actuation cable 3f and the manual actuation element correspond to components 3 and 1 as have been explained above with the aid of Figures 1 to 3, including any variants.

The actuation cable 3 of the biopsy forceps 1 explained with the aid of Figures 1 to 3 is flexible. In order to be able also to insert the biopsy forceps through narrow bends or in a targeted manner over branchings of the vascular system of the patient, the curvature of the end region of the actuation cable adjacent to the forceps tool of the biopsy forceps can preferably be adjusted from the manual actuation element. Figure 10 shows a detail diagram similar to Figure 2 of a forceps tool 5g which is connected via a actuation cable 3g to a manual actuation element (not shown in more detail). The forceps tool 5g corresponds to the forceps tool 5 explained with the aid of Figure 2. For explanation of its components 21 g, 25g to 33g and 49g, reference is made to the explanations of Figure 2.

In contrast to the biopsy forceps of Figure 2, the cable sheath 13g of the actuation cable 3g comprises two lumina 65, 67 separated from one another by an intermediate wall 63, at least one of which and here both lumina 65, 67 run eccentrically to the central axis 39g of the cable sheath 13g along the actuation cable 3g. The cable core 15g which can be displaced longitudinally by means of the actuation element and by means of which the forceps tool 5g can be opened or closed in the manner explained with the aid of Figures 1 to 3 runs in the lumen 65. The lumen 65 can also run centrically to the central axis 39g.

A further cable core 69 is arranged in the lumen 67, the end 71 of which cable core 69 adjacent to the forceps tool 5g is fixed eccentrically to the central axis 39g on the forceps base 21 g. The cable core 69 is otherwise freely displaceable in the lumen 67 and can be loaded with tension by the manual actuation element via a handling unit (not shown in more detail). Because of the eccentricity with which the end 71 of the cable core 69 is fixed on the tool base 21 g, the actuation cable 3g curves in a region immediately adjacent to the forceps tool 5g. The angle of curvature is determined by the displacement path around which the cable core 69 is moved on the manual actuation element relative to the cable sheath 13g. It goes without saying that the end 71 of the cable core 69 close to the forceps base 21 g can also be fixed directly, but eccentrically to the central axis 39g, on the cable sheath 13g.

The handling unit connected to the cable core 69 for adjusting the curvature can have the form of a finger grip, as is shown at 19 in Figure 1. Expediently, however, the handling unit can be stopped or is constructed as a self-limiting gear, for example in the form of an adjusting screw or the like, in order to facilitate operation. In Figure 10, the opening plane of the forceps fingers 29g runs in the plane of the lumina 65, 67. It goes without saying that the opening plane of the forceps fingers 29g can also be displaced at an angle with respect to the plane of the lumina 65, 67 around the central axis 39g.

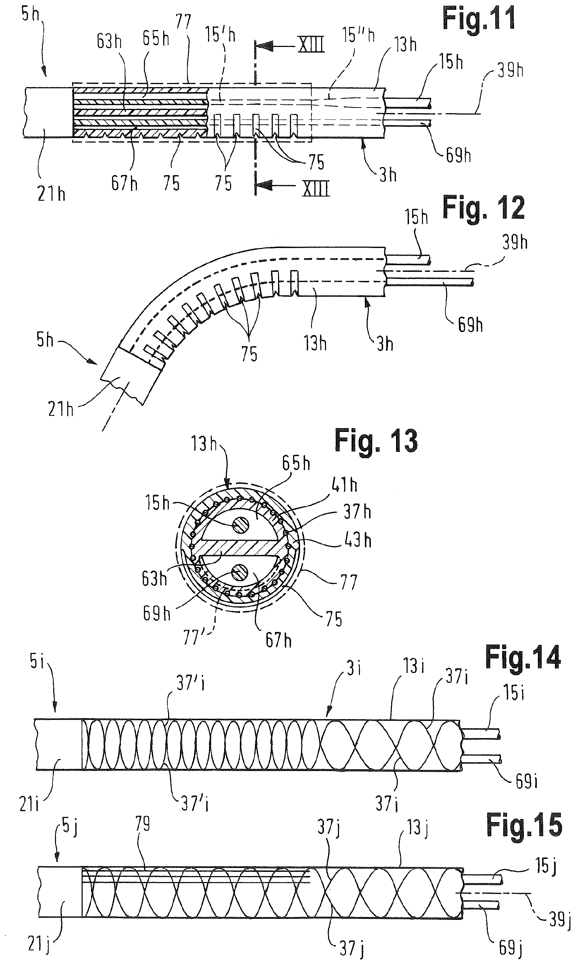

The cable sheath 13g, like the cable cores 15g and 69, are constructed according to the explanations for the cable sheath 13 and the cable core 15 of Figures 1 to 3, and in particular are reinforced by reinforcing threads in each case embedded in a plastics matrix. The intermediate wall 63 is connected here integrally with the cable sheath 15g, as is explained in still more detail below for the embodiment of Figure 13.

It goes without saying that the forceps tool 5g can also be replaced by other surgical tools, as explained, for example, in Figures 4 to 9.

Inhomogeneities in the cable sheath 13g can mean that the actuation cable 3g not only curves in the curvature region but also winds around the central axis 39g. Furthermore, it is desirable for the tensile force to be exerted on the further cable core 69 to adjust the curvature to be comparatively low, since the further cable core 69 loads the cable sheath 13g radially during curving of the actuation cable and may damage the inner wall of the lumen 67. Figures 11 to 13 show a variant of a surgical instrument of the type explained above, in which the winding behaviour is improved and which can be curved with a comparatively low tensile load.

The forceps tool 5h and the actuation cable 3h correspond in construction and mode of action to the embodiment according to Figure 10, including the variants explained there. In contrast to the cable sheath 13g, the cable sheath 13h is provided, in its region adjacent to the tool base 21 h and intended for the controllable curvature, with a plurality of groove-like recesses 75 arranged along the central axis 39h at intervals from one another, which lessen the material cross-section of the cable sheath 13h and accordingly reduce the flexural resistance of the cable sheath 13h. The recesses 75 are arranged on the inside of the bend of the region to be curved and extend in cross-sectional planes perpendicular to the axis over about half the circumference of the outer sheath of the cable sheath 13h, as is to be seen best from Figure 13. The sheath surface lying on the outside of the curvature is free from recesses and stabilises the cable sheath 13h against twisting. The intermediate wall 63h extends substantially perpendicular to the plane of curvature and in this manner likewise contributes towards stabilisation.

The cable sheath 13h comprises, as has already been explained with the aid of Figure 3, an inner sheath layer 41 h, an outer sheath layer 43h and reinforcing threads 37h between these layers. For explanation of the construction and the mode of action including any variants, reference is made to the description of Figures 1 to 3. The intermediate wall 63h is formed integrally, for example by extrusion, on the inner sheath layer 41h.

The recesses 75 are formed in the outer surface of the outer sheath layer 43h and, as shown at 77 in Figures 11 and 13, can be sheathed with a flexible plastic tube 77 to smooth the outer surface of the cable sheath 13h. Alternatively, the recesses, as indicated at 77' in Figure 13, are also formed in the inner sheath layer 41 h, expediently on the outer surface thereof. The recesses can be produced by any desired process, for example by removal of material or also by compression moulding or plastic deformation or the like.

The cable core 15h also contributes towards stiffening of the curvature region of the actuation cable 13h. In order to reduce the flexural rigidity of the curvature region, as indicated at 15'h in Figure 11, the diameter of the cable core 15h is chosen to be smaller in the region intended for the curvature than outside the region up to the manual actuation element. In the transition region between the thinner section 15'h and the thicker section of the cable core 15h, the cable core 15h tapers conically up to the forceps tool 5h, as is indicated at 15'h. It goes without saying that the diameter of the cable core 69h can also be graduated in a corresponding manner.

The reinforcements of the cable jackets explained above which are coiled in opposite directions can have a uniform pitch over the entire length of the actuation cable. As in the embodiment indicated in diagram form in Figure 14, however, in the region which is immediately adjacent to the forceps tool 5i and is provided for the controllable curvature of the actuation cable 3i, the pitch of the reinforcing threads 37i coiled in opposite directions, as indicated at 37'i, is coiled with a lower pitch than outside this region up to the manual actuation element. Because of the lower pitch height, the cable sheath 13i is more flexible in the curvature region than outside this region and can nevertheless be produced without points of impact. It goes without saying that the mode of arrangement of the reinforcing threads can also be employed in the embodiment of Figures 10 to 13, but also in surgical instruments without a controllable curvature region such as, for example, the instruments of Fig. 1 to 8.

A further variant which likewise can be employed in the embodiments of Figures 11 to 14 is shown in Figure 15. In this embodiment, in addition to the reinforcing threads 37j of the cable sheath 13j which are coiled in opposite directions, a reinforcement 79 which stiffens the cable sheath on the outside of the curvature compared with the inside of the curvature is arranged in the region which is adjacent to the forceps tool 5j and is provided for the curvature. The reinforcement 79 can be a plurality of longitudinally running reinforcing threads, but also an elongated flexible reinforcing sheet. The reinforcement 79 improves the twisting rigidity of the curvature region. Instead of or also in addition to the reinforcement 79, it can also be envisaged that the outside of the curvature of the cable sheath is made of a more rigid material than the inside of the curvature. Such a cable sheath can be produced, for example, by coextrusion or pultrusion.

## Claims

1. Surgical instrument, comprising
- a surgical tool (5) having a tool base (21) and a tool element (29) movably guided relative to the tool base (21),
- a manual actuation element (1) for moving the tool element (29) relative to the tool base (21), and
- an elongated, flexible actuation cable (13) which connects the manual actuation element (1) to the surgical tool (5) and has a tubular, flexible cable sheath (13) connected to the tool base (21) and a flexible cable core (15) which is guided displaceably in the cable sheath (13) and is connected to the tool element (29) for movement thereof relative to the tool base,
wherein at least the tool element (29) of the surgical tool (5), but in particular also the tool base (21) thereof, is made substantially of a non-ferromagnetic or substantially non-ferromagnetic metal or of ceramic or of plastic,
and wherein the cable sheath (13) and the cable core (15) are each made of an electrically non-conducting or substantially non-conducting composite material in which a plurality of reinforcing threads (37) are embedded in a plastics matrix,
**characterised in that**
the reinforcing threads (37) of the cable core (15) run substantially parallel to the longitudinal direction of the cable core (15), and
the composite material of the cable sheath (13) comprises reinforcing threads (37) coiled in opposite directions around the central axis (39) thereof.

2. Surgical instrument according to claim 1, **characterised in that** the cable sheath (13) comprises a tubular, inner sheath layer (41) of electrically non-conducting or substantially non-conducting plastics material and a tubular outer sheath layer (43), surrounding the inner sheath layer (41), of electrically non-conducting or substantially non-conducting plastics material, the reinforcing threads (37) being arranged between the inner (41) and the outer (43) sheath layer, wherein the inner sheath layer (41) and the outer sheath layer (43) are formed from plastics materials having a hardness and/or flexural elasticity which differ from one another.

3. Surgical instrument according to claim 2, **characterised in that** the inner sheath layer (41) is harder or more rigid than the outer sheath layer (43).

4. Surgical instrument, comprising
- a surgical tool (5) having a tool base (21) and a tool element (29) movably guided relative to the tool base (21),
- a manual actuation element (1) for moving the tool element (29) relative to the tool base (21), and
- an elongated, flexible actuation cable (13) which connects the manual actuation element (1) to the surgical tool (5) and has a tubular, flexible cable sheath (13) connected to the tool base (21) and a flexible cable core (15) which is guided displaceably in the cable sheath (13) and is connected to the tool element (29) for movement thereof relative to the tool base,
wherein at least the tool element (29) of the surgical tool (5), but in particular also the tool base (21) thereof, is made substantially of a non-ferromagnetic or substantially non-ferromagnetic metal or of ceramic or of plastic,
and wherein the cable sheath (13) and the cable core (15) are each made of an electrically non-conducting or substantially non-conducting composite material in which a plurality of reinforcing threads (37) are embedded in a plastics matrix,
**characterised in that**
the reinforcing threads (37) of the cable core (15) run substantially parallel to the longitudinal direction of the cable core (15), and
**in that** the cable sheath (13) comprises a tubular, inner sheath layer (41) of electrically non-conducting or substantially non-conducting plastics material and a tubular outer sheath layer (43), surrounding the inner sheath layer (41), of electrically non-conducting or substantially non-conducting plastics material, the reinforcing threads (37) being arranged between the inner (41) and the outer (43) sheath layer, wherein the inner sheath layer (41) and the outer sheath layer (43) are formed from plastics materials having a hardness and/or flexural elasticity which differ from one another, such that the inner sheath layer (41) is harder or more rigid than the outer sheath layer (43).

5. Surgical instrument according to any of claims 1 to 4, **characterised in that** at least the tool element (29) or the tool element (29) including the tool base (21) is made substantially of a paramagnetic or diamagnetic material, in particular titanium or platinum or gold or silver or copper or aluminium or an alloy based on at least one of these metals.

6. Surgical instrument according to any of claims 1 to 5, **characterised in that** the overall dimensions of the surgical tool (5) and/or of the manual actuation element (1), where this is made of electrically conducting material, in particular metal, are less than 10 cm, in particular less than 6 cm, preferably less than 3 cm.

7. Surgical instrument according to any of claims 1 to 6 **characterised in that** the composite material of the cable sheath (13) comprises a woven structure of reinforcing threads (37), in particular in the form of a woven tube.

8. Surgical instrument according to any of claims 1 to 7, **characterised in that**
a) the reinforcing threads (37) of the cable core (15) are in each case longer than 3 cm, in particular extend in one piece over the entire length of the cable core (15), and/or
b) the reinforcing threads (37) of the composite material of the cable sheath (13) and/or of the cable core (15) are constructed as aramid fibres or glass fibres or polyester fibres or ceramic fibres or fibres of liquid crystal polymer, and/or
c) the composite material of the cable sheath (13) and/or of the cable core (15) comprises a mixture of reinforcing threads of different material, in particular of glass fibres and aramid fibres.

9. Surgical instrument according to any of claims 1 to 8, **characterised in that** the plastics matrix of the composite material of the cable core (15)
a) comprises at least one plastic from the group of polyamides, in particular PA 6 or PA66, and/or the group of polyimides (PI) and/or polyetherimides (PEI), and/or the group of polyamide-imides (PAI), and/or the group of polyaryletherketones (PAEK), and/or polyetheretherketones (PEEK) and/or of polyetherketones (PEK), and/or the group of polyphenylene sulphides (PPS), and/or the group of polysulfones (PSU) in particular polyarylsulfones and/or of polyphenylsulfones (PPSU) and/or of polyethersulfones, and/or the group of liquid crystal polymers (LCP), and/or
b) has a ball indentation hardness of at least 120 MPa and/or an E modulus of at least 2,000 MPa and/or a yield stress of at least 60 MPa.

10. Surgical instrument according to any of claims 1 to 9, **characterised in that** the plastics matrix of the composite material of the cable sheath (13) comprises at least one of the plastics: polyamide (PA), polyethylene (PE), polypropylene (PP), polyurethane (PU), polytetrafluoroethylene (PTFE), perfluoroethylene-propylene (FEP), perfluoroalkoxyalkane (PFA) and polyether block amide (PEBAX).

11. Surgical instrument according to any of claims 1 to 10, **characterised in that** the cable sheath (13) and/or the cable core (15) is/are coated or doped at least in regions with marking material, in particular in the form of particles, of a material which has a susceptibility which differs from water, or carries at least one metal ring.

12. Surgical instrument according to any of claims 1 to 11, **characterised in that** the manual actuation element (1) is made of a non-magnetic, electrically non-conducting material, in particular plastic, at least in its manually accessible outer surfaces.

13. Surgical instrument according to any of claims 1 to 12, **characterised in that** the surgical tool is constructed as a holding tool (5) having a gripping device (29) which forms the tool element and is guided such that it can move relative to the tool base (21), the manual actuation element (1) being connected via the cable core (15) to the gripping device (29) for opening and closing the holding tool (5), in particular wherein the holding tool is constructed as a forceps tool (5) on the tool base of which, serving as the forceps base (21), at least two forceps fingers (29) forming the gripping device are movably guided and are connected to the cable core (15) for opening and closing the forceps tool (5),
and further in particular for generation of different MRT artefacts, at least one of the forceps fingers (29) is made of a different material to at least one other of the forceps fingers (29) and/or is coated and/or doped with a material which modifies the MRT artefacts of the at least one forceps finger (29).

14. Surgical instrument according to any of claims 1 to 13, **characterised in that** the tool element which can move relative to the tool base (21c) is constructed as a collecting loop (51) or as a collecting basket (53) or as a coupling hook (57).

15. Surgical instrument according to any of claims 1 to 14, **characterised in that**, in a region adjacent to the surgical tool (5i), the reinforcing threads (37i) of the cable sheath (13i) are coiled around the central axis (39i) with a smaller pitch than in a region lying between this region and the manual actuation element.

16. Surgical instrument according to any of claims 1 to 15, **characterised in that** a further, flexible cable core (69, 69h-j) of electrically non-conducting or substantially non-conducting material is guided displaceably in the cable sheath (13g-j), the end of which cable core adjacent to the tool base (5g-j) is fixed eccentrically to a central axis (39g-j) of the cable sheath (13g-j) on the tool base (5g-j) or close to the tool base (5g-j) on the cable sheath (13g-j).

17. Surgical instrument according to claim 16, **characterised in that** for formation of a region of controllable curvature, the cable sheath (13h, j), in a section of its length adjacent to the tool base (21 h, j), has non-uniform flexurally elastic properties transversely to the central axis (39h, j) and, viewed in the circumferential direction, is more flexible towards the eccentrically fixed end of the further cable core than outside this region.

18. Surgical instrument according to claim 17, **characterised in that** in the region of controllable curvature the cable sheath (13h) has, on the side lying towards the eccentric end of the further cable core (69h) with respect to the central axis (39h), a plurality of recesses (75) arranged at intervals from one another in the direction of the central axis (39h), in particular grooves or cavities extending in the circumferential direction over a part-length of the circumference, in particular wherein the recesses (75) are provided on the outer circumference of the cable sheath (13h), further in particular if the cable sheath (13h) is sheathed with a flexible protective tube (77) at least in the region of controllable curvature.

19. Surgical instrument according to any of claims 17 to 18, **characterised in that**
a) in the region of controllable curvature, on the side of the central axis (39g) lying radially to the eccentrically fixed end (71) of the further cable core (69), the cable sheath (13g) is made of a more flexible material at least in a part-region than at least in a part-region on the diametrally opposite side, and/or
b) on the side of the central axis (39j) facing radially away from the eccentrically fixed end of the further cable core (69j), the cable sheath (13j) is provided, in the region of controllable curvature, with a reinforcement (79) extended in the direction of the central axis (39j), and/or
c) in the region of controllable curvature, the cable core (15h) and/or the further cable core (69h) has/have a smaller diameter than on the side of the cable sheath (13h) away from the tool base (21 h).

## Patentansprüche

1. Chirurgisches Instrument, welches aufweist:
- ein chirurgisches Werkzeug (5) mit einer Werkzeugbasis (21) und einem Werkzeugelement (29), das relativ zu der Werkzeugbasis (21) bewegbar geführt ist,
- ein manuelles Betätigungselement (1) zum Bewegen des Werkzeugelements (29) relativ zur Werkzeugbasis (21), und
- ein längliches flexibles Betätigungskabel (13), das das manuelle Betätigungselement (1) mit dem chirurgischen Werkzeug (5) verbindet und eine rohrförmige flexible Kabelhülle (13), die mit der Werkzeugbasis (21) verbunden ist, sowie einen flexiblen Kabelkern (15), der in der Kabelhülle (13) verschiebbar geführt ist und mit dem Werkzeugelement (29) zu dessen Bewegung relativ zur Werkzeugbasis verbunden ist, aufweist,
wobei zumindest das Werkzeugelement (29) des chirurgischen Werkzeugs (5), aber insbesondere auch seine Werkzeugbasis (21), im Wesentlichen aus nicht-ferromagnetischem oder im Wesentlichen nicht-ferromagnetischem Metall oder aus Keramik oder aus Kunststoff hergestellt ist,
und wobei die Kabelhülle (13) und der Kabelkern (15) jeweils aus einem elektrisch nicht-leitenden oder im Wesentlichen nicht-leitenden Kompositmaterial hergestellt sind, in dem eine Mehrzahl von Verstärkungsfäden (37) in eine Kunststoffmatrix eingebettet sind, **dadurch gekennzeichnet, dass**
die Verstärkungsfäden (37) des Kabelkerns (15) im Wesentlichen parallel zur Längsrichtung des Kabelkerns (15) verlaufen, und
das Kompositmaterial der Kabelhülle (13) Verstärkungsfäden (37) aufweist, die um deren Mittelachse (39) in entgegengesetzte Richtungen gewickelt sind.

2. Chirurgisches Instrument nach Anspruch 1, **dadurch gekennzeichnet, dass** die Kabelhülle (13) eine rohrförmige innere Hüllenschicht (41) aus elektrisch nicht-leitendem oder im Wesentlichen nicht-leitendem Kunststoffmaterial sowie eine die innere Hüllenschicht (41) umgebende rohrförmige äußere Hüllenschicht (43) aus elektrisch nicht-leitendem oder im Wesentlichen nicht-leitendem Kunststoffmaterial aufweist, wobei die Verstärkungsfäden (37) zwischen der inneren (41) und der äußeren (43) Hüllenschicht angeordnet sind, wobei die innere Hüllenschicht (41) und die äußere Hüllenschicht (43) aus Kunststoffmaterialien hergestellt sind, die eine Härte und/oder Biegeelastizität haben, die voneinander unterschiedlich sind.

3. Chirurgisches Instrument nach Anspruch 2, **dadurch gekennzeichnet, dass** die innere Hüllenschicht (41) härter oder steifer als die äußeren Hüllenschicht (43) ist.

4. Chirurgisches Instrument, welches aufweist:
- ein chirurgisches Werkzeug (5) mit einer Werkzeugbasis (21) und einem Werkzeugelement (29), das relativ zu der Werkzeugbasis (21) bewegbar geführt ist,
- ein manuelles Betätigungselement (1) zum Bewegen des Werkzeugelements (29) relativ zur Werkzeugbasis (21), und
- ein längliches flexibles Betätigungskabel (13), das das manuelle Betätigungselement (1) mit dem chirurgischen Werkzeug (5) verbindet und eine rohrförmige flexible Kabelhülle (13), die mit der Werkzeugbasis (21) verbunden ist, sowie einen flexiblen Kabelkern (15), der in der Kabelhülle (13) verschiebbar geführt ist und mit dem Werkzeugelement (29) zu dessen Bewegung relativ zur Werkzeugbasis verbunden ist, aufweist,
wobei zumindest das Werkzeugelement (29) des chirurgischen Werkzeugs (5), aber insbesondere auch seine Werkzeugbasis (21), im Wesentlichen aus nicht-ferromagnetischem oder im Wesentlichen nicht-ferromagnetischem Metall oder aus Keramik oder aus Kunststoff hergestellt ist,
und wobei die Kabelhülle (13) und der Kabelkern (15) jeweils aus einem elektrisch nicht-leitenden oder im Wesentlichen nicht-leitenden Kompositmaterial hergestellt sind, in dem eine Mehrzahl von Verstärkungsfäden (37) in eine Kunststoffmatrix eingebettet sind, **dadurch gekennzeichnet, dass**
die Verstärkungsfäden (37) des Kabelkerns (15) im Wesentlichen parallel zur Längsrichtung des Kabelkerns (15) verlaufen, und
dass die Kabelhülle (13) eine rohrförmige innere Hüllenschicht (41) aus elektrisch nicht-leitendem oder im Wesentlichen nicht-leitendem Kunststoffmaterial sowie eine die innere Hüllenschicht (41) umgebende rohrförmige äußere Hüllenschicht (43) aus elektrisch nicht-leitendem oder im Wesentlichen nicht-leitendem Kunststoffmaterial aufweist, wobei die Verstärkungsfäden (37) zwischen der inneren (41) und der äußeren (43) Hüllenschicht angeordnet sind, wobei die innere Hüllenschicht (41) und die äußere Hüllenschicht (43) aus Kunststoffmaterialien gebildet sind, deren eine Härte und/oder Biegeelastizität voneinander unterschiedlich sind, so dass die innere Hüllenschicht (41) härter oder steifer als die äußeren Hüllenschicht (43) ist.

5. Chirurgisches Instrument nach einem der Ansprüche 1 bis 4, **dadurch gekennzeichnet, dass** zumindest das Werkzeugelement (29) oder das Werkzeugelement (29) einschließlich der Werkzeugbasis (21) im Wesentlichen aus paramagnetischem oder diamagnetischem Material hergestellt ist, insbesondere Titan oder Platin oder Gold oder Silber oder Kupfer oder Aluminium oder einer Legierung basierend auf zumindest einem dieser Metalle.

6. Chirurgisches Instrument nach einem der Ansprüche 1 bis 5, **dadurch gekennzeichnet, dass** die Gesamtabmessungen des chirurgischen Werkzeugs (5) und/oder des manuellen Betätigungselements (1), wo es aus elektrisch leitendem Material, insbesondere Metall hergestellt ist, kleiner als 10 cm, insbesondere kleiner als 6 cm, bevorzugt kleiner als 3 cm sind.

7. Chirurgisches Instrument nach einem der Ansprüche 1 bis 6, **dadurch gekennzeichnet, dass** das Kompositmaterial der Kabelhülle (13) eine gewobene Struktur aus Verstärkungsfäden (37) aufweist, insbesondere in der Form eines gewobenen Rohrs.

8. Chirurgisches Instrument nach einem der Ansprüche 1 bis 7, **dadurch gekennzeichnet, dass**
a) die Verstärkungsfäden (37) des Kabelkerns (15) in jedem Fall länger als 3 cm sind, sich insbesondere in einem Stück über die Gesamtlänge des Kabelkerns (15) erstrecken, und/oder
b) die Verstärkungsfäden (37) des Kompositmaterials der Kabelhülle (13) und/oder des Kabelkerns (15) aus Aramidfasern oder Glasfasern oder Polyesterfasern oder Keramikfasern oder Fasern aus Flüssigkristall-Polymer aufgebaut sind, und/oder
c) das Kompositmaterial der Kabelhülle (13) und/oder des Kabelkerns (15) ein Gemisch von Verstärkungsfäden aus unterschiedlichem Material aufweist, insbesondere Glasfasern und Aramidfasern.

9. Chirurgisches Instrument nach einem der Ansprüche 1 bis 8, **dadurch gekennzeichnet, dass** die Kunststoffmatrix des Kompositmaterials des Kabelkerns (15)
a) weingstens einen Kunststoff aus der Gruppe von Polyamiden aufweist, insbesondere PA 6 oder PA66, und/oder der Gruppe von Polyimiden (PI) und/oder Polyetherimiden (PEI), und/oder der Gruppe von Polyamid-Imiden (PAI), und/oder der Gruppe von Polyacryletherketonen (PAEK), und/oder Polyetheretherketonen (PEEK), und/oder Polyetherketonen (PEK), und/oder der Gruppe von Polyphenylensulfiden (PPS), und/oder der Gruppen von Polysulfonen (PSU), insbesondere Polyacrylsulfonen und/oder Polyphenylsulfonen (PPSU) und/oder Polyethersulfonen, und/oder der Gruppe von Flüssigkristallpolymeren (LCP), und/oder
b) eine Kugeleindruckhärte von wenigstens 120 MPa und/oder einen E-Modul von wenistens 2.000 MPa und/oder eine Streckgrenze von wenigstens 60 MPa aufweist.

10. Chirurgisches Instrument nach einem der Ansprüche 1 bis 9, **dadurch gekennzeichnet, dass** die Kunststoffmatrix des Kompositmaterials der Kabelhülle (13) wenigstens eines der Kunststoffe aufweist: Polyamid (PA), Polyethylen (PE), Polypropylen (PP), Polyurethan (PU), Polytetrafluorethylen (PTFE), Perfluorethylenpropylen (FEP), Perfluoralkoxyalkan (PFA) und Polyetherblockamid (PEBAX).

11. Chirurgisches Instrument nach einem der Ansprüche 1 bis 10, **dadurch gekennzeichnet, dass** die Kabelhülle (13) und/oder der Kabelkern (15) zumindest in Bereichen Markierungsmaterial, insbesondere in der Form von Partikeln, eines Materials beschichtet oder dotiert ist/sind, das eine Empfindlichkeit hat, die sich von Wasser unterscheidet, oder wenigstens einen Metallring trägt.

12. Chirurgisches Instrument nach einem der Ansprüche 1 bis 11, **dadurch gekennzeichnet, dass** das manuelle Betätigungselement (1), zumindest in seinen manuell zugänglichen Außenoberflächen, aus nichtmagnetischem elektrisch nicht-leitendem Material, insbesondere Kunststoff, hergestellt ist.

13. Chirurgisches Instrument nach einem der Ansprüche 1 bis 12, **dadurch gekennzeichnet, dass** das chirurgische Werkzeug als Haltewerkzeug (5) mit einer Greifvorrichtung (29) aufgebaut ist, die das Werkzeugelement bildet und derart geführt ist, dass sie sich relativ zur Werkzeugbasis (21) bewegen kann, wobei das manuelle Betätigungselement (1) über den Kabelkern (15) mit der Greifvorrichtung (29) Öffnen und Schließen des Haltewerkzeugs (5) verbunden ist, wobei das Haltewerkzeug insbesondere als Zangenwerkzeug (5) aufgebaut ist, an dessen Werkzeugbasis, die als die Zangenbasis (21) dient, wenigstens zwei Zangenfinger (29), welche die Greifvorrichtung bilden, beweglich geführt sind und mit dem Kabelkern (15) verbunden sind, um das Zangenwerkzeug (5) zu öffnen und zu schließen,
und ferner insbesondere zum Erzeugen von unterschiedlichen MRT-Artefakten, wenigstens einer der Zangenfinger (29) aus einem anderen Material als wenigstens ein anderer der Zangenfinger (29) hergestellt ist und/oder mit einem Material, das die MRT-Artefakte des wenigstens einen Zangenfingers (29) modifiziert, beschichtet und/oder dotiert ist.

14. Chirurgisches Instrument nach einem der Ansprüche 1 bis 13, **dadurch gekennzeichnet, dass** das Werkzeugelement, das sich relativ zur Werkzeugbasis (21 c) bewegen kann, als Sammelschleife (51) oder als Sammelkorb (53) oder als Kupplungshaken (57) aufgebaut ist.

15. Chirurgisches Instrument nach einem der Ansprüche 1 bis 14, **dadurch gekennzeichnet, dass** in einem Bereich benachbart dem chirurgischem Werkzeug (5i), die Verstärkungsfäden (37i) der Kabelhülle (13i) um die Mittelachse (39i) mit einer kleineren Steigung herumgewickelt sind als in einem Bereich, der zwischen diesem Bereich und dem manuellen Betätigungselement liegt.

16. Chirurgisches Instrument nach einem der Ansprüche 1 bis 15, **dadurch gekennzeichnet, dass** ein weiterer flexibler Kabelkern (69, 69h-j) aus elektrisch nicht-leitendem oder im Wesentlichen nicht-leitendem Material in der Kabelhülle (13g-j) verschiebbar geführt ist, wobei das der Werkzeugbasis (5g-j) benachbarte Ende dieses Kabelkerns exzentrisch einer Mittelachse (39g-j) der Kabelhülle (13g-j) an der Werkzeugbasis (5g-j) oder benachbart der Werkzeugbasis (5g-j) an der Kabelhülle (13g-j) befestigt ist.

17. Chirurgisches Instrument nach Anspruch 16, **dadurch gekennzeichnet, dass** zur Bildung eines Bereichs einer steuerbaren Krümmung die Kabelhülle (13h, j), in einem Abschnitt seiner Länge benachbart der Werkzeugbasis (21 h, j), ungleichmäßige biegeelastische Eigenschaften quer zur Mittelachse (39h, j) hat, und, bei Betrachtung in der Umfangsrichtung, zu dem exzentrisch fixierten Ende des weiteren Kabelkerns hin flexibler ist als außerhalb dieses Bereichs.

18. Chirurgisches Instrument nach Anspruch 17, **dadurch gekennzeichnet, dass** in dem Bereich der steuerbaren Krümmung die Kabelhülle (13h) an der zum exzentrischen Ende des weiteren Kabelkerns (69h) liegenden Seite in Bezug auf die Mittelachse (39h), eine Mehrzahl von Vertiefungen (75) aufweist, die mit Intervallen voneinander in Richtung der Mittelachse (39h) angeordnet sind, insbesondere Nuten oder Ausnehmungen, die sich in der Umfangsrichtung über eine Teillänge des Umfangs hinweg erstrecken, wobei insbesondere die Vertiefungen (75) am Außenumfang der Kabehülle (13h) vorgesehen sind, ferner insbesondere, wenn die Kabelhülle (13h) zumindest im Bereich der steuerbaren Krümmung mit einem flexiblen Schutzrohr (77) umhüllt ist.

19. Chirurgisches Instrument nach einem der Ansprüche 17 bis 18, **dadurch gekennzeichnet, dass**
a) in dem Bereich der steuerbaren Krümmung an der Seite der Mittelachse (39g), die radial zum exzentrisch fixierten Ende (71) des weiteren Kabelkerns (69) liegt, die Kabelhülle (13g) zumindest in einem Teilbereich aus flexiblerem Material hergestellt ist als zumindest in einem Teilbereich an der diametral entgegengesetzten Seite, und/oder
b) an der Seite der Mittelachse (39j), die von dem exzentrisch fixierten Ende des weiteren Kabelkerns (69j) radial weg weist, die Kabelhülle (13j) im Bereich der steuerbaren Kurve mit einer Verstärkung (79) versehen ist, die sich in der Richtung der Mittelachse (39j) erstreckt, und/oder
c) in dem Bereich der steuerbaren Krümmung der Kabelkern (15h) und/oder der weitere Kabelkern (69h) einen kleineren Durchmesser als an der von der Werkzeugbasis (21h) entfernten Seite der Kabelhülle (13h) aufweist/aufweisen.

## Revendications

1. Instrument chirurgical, comprenant
- un outil chirurgical (5) ayant une base d'outil (21) et un élément d'outil (29) guidé de manière mobile par rapport à la base d'outil (21),
- un élément d'actionnement manuel (1) pour bouger l'élément d'outil (29) par rapport à la base d'outil (21), et
- un câble d'actionnement flexible allongé (13) qui relie l'élément d'actionnement manuel (1) à l'outil chirurgical (5) et a une gaine de câble flexible tubulaire (13) reliée à la base d'outil (21) et une âme de câble flexible (15) qui est guidée de manière à pouvoir se déplacer dans la gaine de câble (13) et est reliée à l'élément d'outil (29) pour un mouvement de celui-ci par rapport à la base d'outil,
dans lequel au moins l'élément d'outil (29) de l'outil chirurgical (5), mais en particulier aussi la base d'outil (21) de celui-ci, est constitué(e) sensiblement d'un métal non ferromagnétique ou sensiblement non ferromagnétique ou de céramique ou de plastique,
et dans lequel la gaine de câble (13) et l'âme de câble (15) sont chacune constituées d'un matériau composite électriquement non conducteur ou sensiblement non conducteur dans lequel une pluralité de fils de renfort (37) sont logés dans une matrice plastique,
**caractérisé en ce que**
les fils de renfort (37) de l'âme de câble (15) sont sensiblement parallèles à la direction longitudinale de l'âme de câble (15), et
le matériau composite de la gaine de câble (13) comprend des fils de renfort (37) enroulés dans des directions opposées autour de l'axe central (39) de celle-ci.

2. Instrument chirurgical selon la revendication 1, **caractérisé en ce que** la gaine de câble (13) comprend une couche de gaine interne tubulaire (41) de matériau plastique électriquement non conducteur ou sensiblement non conducteur et une couche de gaine externe tubulaire (43), entourant la couche de gaine interne (41), de matériau plastique électriquement non conducteur ou sensiblement non conducteur, les fils de renfort (37) étant agencés entre la couche de gaine interne (41) et la couche de gaine externe (43), dans lequel la couche de gaine interne (41) et la couche de gaine externe (43) sont formées de matériaux plastiques ayant une dureté et/ou une élasticité à la flexion qui diffèrent l'une de l'autre.

3. Instrument chirurgical selon la revendication 2, **caractérisé en ce que** la couche de gaine interne (41) est plus dure ou plus rigide que la couche de gaine externe (43).

4. Instrument chirurgical, comprenant
- un outil chirurgical (5) ayant une base d'outil (21) et un élément d'outil (29) guidé de manière mobile par rapport à la base d'outil (21),
- un élément d'actionnement manuel (1) pour bouger l'élément d'outil (29) par rapport à la base d'outil (21), et
- un câble d'actionnement flexible allongé (13) qui relie l'élément d'actionnement manuel (1) à l'outil chirurgical (5) et a une gaine de câble flexible tubulaire (13) reliée à la base d'outil (21) et une âme de câble flexible (15) qui est guidée de manière à pouvoir se déplacer dans la gaine de câble (13) et est reliée à l'élément d'outil (29) pour un mouvement de celui-ci par rapport à la base d'outil,
dans lequel au moins l'élément d'outil (29) de l'outil chirurgical (5), mais en particulier aussi la base d'outil (21) de celui-ci, est constitué(e) sensiblement d'un métal non ferromagnétique ou sensiblement non ferromagnétique ou de céramique ou de plastique,
et dans lequel la gaine de câble (13) et l'âme de câble (15) sont chacune constituées d'un matériau composite électriquement non conducteur ou sensiblement non conducteur dans lequel une pluralité de fils de renfort (37) sont logés dans une matrice plastique,
**caractérisé en ce que**
les fils de renfort (37) de l'âme de câble (15) sont sensiblement parallèles à la direction longitudinale de l'âme de câble (15), et
**en ce que** la gaine de câble (13) comprend une couche de gaine interne tubulaire (41) de matériau plastique électriquement non conducteur ou sensiblement non conducteur et une couche de gaine externe tubulaire (43), entourant la couche de gaine interne (41), de matériau plastique électriquement non conducteur ou sensiblement non conducteur, les fils de renfort (37) étant agencés entre les couches de gaine interne (41) et externe (43), dans lequel la couche de gaine interne (41) et la couche de gaine externe (43) sont formées de matériaux plastiques ayant une dureté et/ou une élasticité à la flexion qui diffèrent l'une de l'autre, de telle sorte que la couche de gaine interne (41) est plus dure ou plus rigide que la couche de gaine externe (43).

5. Instrument chirurgical selon l'une quelconque des revendications 1 à 4, **caractérisé en ce qu'**au moins l'élément d'outil (29) ou l'élément d'outil (29) comportant la base d'outil (21) est constitué sensiblement d'un matériau paramagnétique ou diamagnétique, en particulier de titane ou de platine ou d'or ou d'argent ou de cuivre ou d'aluminium ou d'un alliage à base d'au moins l'un de ces métaux.

6. Instrument chirurgical selon l'une quelconque des revendications 1 à 5, **caractérisé en ce que** les dimensions globales de l'outil chirurgical (5) et/ou de l'élément d'actionnement manuel (1), où celui-ci est constitué de matériau électriquement conducteur, en particulier de métal, sont inférieures à 10 cm, en particulier inférieures à 6 cm, de préférence, inférieures à 3 cm.

7. Instrument chirurgical selon l'une quelconque des revendications 1 à 6, **caractérisé en ce que** le matériau composite de la gaine de câble (13) comprend une structure tissée de fils de renfort (37), en particulier sous la forme d'un tube tissé.

8. Instrument chirurgical selon l'une quelconque des revendications 1 à 7, **caractérisé en ce que**
a) les fils de renfort (37) de l'âme de câble (15) sont dans chaque cas plus longs que 3 cm, en particulier s'étendent d'un seul tenant sur l'ensemble de la longueur de l'âme de câble (15), et/ou
b) les fils de renfort (37) du matériau composite de la gaine de câble (13) et/ou de l'âme de câble (15) sont construits comme des fibres d'aramide ou des fibres de verre ou des fibres de polyester ou des fibres de céramique ou des fibres de polymère à cristaux liquides, et/ou
c) le matériau composite de la gaine de câble (13) et/ou de l'âme de câble (15) comprend un mélange de fils de renfort de matériau différent, en particulier de fibres de verre et de fibres d'aramide.

9. Instrument chirurgical selon l'une quelconque des revendications 1 à 8, **caractérisé en ce que** la matrice plastique du matériau composite de l'âme de câble (15)
a) comprend au moins un plastique du groupe des polyamides, en particulier PA 6 ou PA66, et/ou du groupe des polyimides (PI) et/ou des polyétherimides (PEI), et/ou du groupe des polyamide-imides (PAI), et/ou du groupe des polyaryléthercétones (PAEK), et/ou polyétheréthercétones (PEEK) et/ou des polyéthercétones (PEK), et/ou du groupe des polyphénylène sulfides (PPS), et/ou du groupe des polysulfones (PSU), en particulier des polyarylsulfones et/ou des polyphénylsulfones (PPSU) et/ou des polyéthersulfones, et/ou du groupe des polymères à cristaux liquides (LCP), et/ou
b) a une dureté à la pénétration à la bille d'au moins 120 MPa et/ou un module E d'au moins 2 000 MPa et/ou une limite d'élasticité d'au moins 60 MPa.

10. Instrument chirurgical selon l'une quelconque des revendications 1 à 9, **caractérisé en ce que** la matrice plastique du matériau composite de la gaine de câble (13) comprend au moins l'un des plastiques suivants : polyamide (PA), polyéthylène (PE), polypropylène (PP), polyuréthane (PU), polytétrafluoroéthylène (PTFE), perfluoroéthylène-propylène (FEP), perfluoroalcoxyalcane (PFA) et polyéther bloc amide (PEBAX).

11. Instrument chirurgical selon l'une quelconque des revendications 1 à 10, **caractérisé en ce que** la gaine de câble (13) et/ou l'âme de câble (15) est/sont revêtue(s) ou dopée(s) au moins dans des régions de matériau de marquage, en particulier sous la forme de particules, d'un matériau qui a une susceptibilité qui diffère de l'eau, ou porte(nt) au moins un anneau métallique.

12. Instrument chirurgical selon l'une quelconque des revendications 1 à 11, **caractérisé en ce que** l'élément d'actionnement manuel (1) est constitué d'un matériau non magnétique électriquement non conducteur, en particulier de plastique, au moins en ses surfaces externes manuellement accessibles.

13. Instrument chirurgical selon l'une quelconque des revendications 1 à 12, **caractérisé en ce que** l'outil chirurgical est construit comme un outil de maintien (5) ayant un dispositif de préhension (29) qui forme l'élément d'outil et est guidé de telle sorte qu'il peut bouger par rapport à la base d'outil (21), l'élément d'actionnement manuel (1) étant relié via l'âme de câble (15) au dispositif de préhension (29) pour ouvrir et fermer l'outil de maintien (5), en particulier dans lequel l'outil de maintien est construit comme un outil formant pince (5) sur la base d'outil dont, servant de base de pince (21), au moins deux doigts de pince (29) formant le dispositif de préhension sont guidés de manière mobile et sont reliés à l'âme de câble (15) pour ouvrir et fermer l'outil de pince (5),
et en outre en particulier pour la génération de différents artéfacts MRT, au moins un des doigts de pince (29) est constitué d'un matériau différent de l'au moins un autre des doigts de pince (29) et/ou est revêtu et/ou dopé d'un matériau qui modifie les artéfacts MRT de l'au moins un doigt de pince (29).

14. Instrument chirurgical selon l'une quelconque des revendications 1 à 13, **caractérisé en ce que** l'élément d'outil qui peut bouger par rapport à la base d'outil (21c) est construit sous la forme d'une boucle de collecte (51) ou sous la forme d'un panier de collecte (53) ou sous la forme d'un crochet de couplage (57).

15. Instrument chirurgical selon l'une quelconque des revendications 1 à 14, **caractérisé en ce que**, dans une région adjacente à l'outil chirurgical (5i), les fils de renfort (37i) de la gaine de câble (13i) sont enroulés autour de l'axe central (39i) avec un pas plus petit que dans une région s'étendant entre cette région et l'élément d'actionnement manuel.

16. Instrument chirurgical selon l'une quelconque des revendications 1 à 15, **caractérisé en ce qu'**une autre âme de câble flexible (69, 69h-j) de matériau électriquement non conducteur ou sensiblement non conducteur est guidée de manière à pouvoir se déplacer dans la gaine de câble (13g-j), l'extrémité de laquelle âme de câble adjacente à la base d'outil (5g-j) étant fixée de manière excentrique à un axe central (39g-j) de la gaine de câble (13g-j) sur la base d'outil (5g-j) ou près de la base d'outil (5g-j) sur la gaine de câble (13g-j).

17. Instrument chirurgical selon la revendication 16, **caractérisé en ce que** pour la formation d'une région de courbure contrôlable, la gaine de câble (13h, j), dans une section de sa longueur adjacente à la base d'outil (21h, j), a des propriétés élastiques non uniformes à la flexion transversalement à l'axe central (39h, j) et, vue dans la direction circonférentielle, est plus flexible vers l'extrémité fixée de manière excentrique de l'autre âme de câble qu'à l'extérieur de cette région.

18. Instrument chirurgical selon la revendication 17, **caractérisé en ce que** dans la région de courbure contrôlable, la gaine de câble (13h) a, sur le côté s'étendant vers l'extrémité excentrique de l'autre âme de câble (69h) par rapport à l'axe central (39h), une pluralité d'évidements (75) agencés à intervalles les uns des autres dans la direction de l'axe central (39h), en particulier des gorges ou cavités s'étendant dans la direction circonférentielle sur une longueur partielle de la circonférence, en particulier dans lequel les évidements (75) sont prévus sur la circonférence externe de la gaine de câble (13h), en outre en particulier si la gaine de câble (13h) est gainée avec un tube protecteur flexible (77) au moins dans la région de courbure contrôlable.

19. Instrument chirurgical selon l'une quelconque des revendications 17 à 18, **caractérisé en ce que**
a) dans la région de courbure contrôlable, sur le côté de l'axe central (39g) s'étendant radialement par rapport à l'extrémité fixée de manière excentrique (71) de l'autre âme de câble (69), la gaine de câble (13g) est constituée d'un matériau plus flexible au moins dans une région partielle que dans une région partielle sur le côté diamétralement opposé, et/ou
b) sur le côté de l'axe central (39j) orienté radialement vers l'opposé de l'extrémité fixée de manière excentrique de l'autre âme de câble (69j), la gaine de câble (13j), est pourvue, dans la région de courbure contrôlable, d'un renfort (79) s'étendant dans la direction de l'axe central (39j), et/ou
c) dans la région de courbure contrôlable, l'âme de câble (15h) et/ou l'autre âme de câble (69h) a/ont un diamètre plus petit que sur le côté de la gaine de câble (13h) vers l'opposé de la base d'outil (21h).
